# EUROPEAN PATENT SPECIFICATION

(11) **EP 3 994 450 B1**
(45) Date of publication and mention of the grant of the patent: **25.02.2026**
(21) Application number: 20742519.0
(22) Date of filing: 30.06.2020
(51) Int. Cl.: G01N 21/552, G01N 33/53, G01N 33/543

(54) **PORTABLE SPR SENSOR FOR DETECTING ANAPHYLAXIS, AND CORRESPONDING METHOD**
TRAGBARER SPR SENSOR ZUR DETEKTION VON UND ANAPHYLAXIE, UND ENTSPRECHENDES VERFAHREN
CAPTEUR SPR PORTABLE POUR DÉTECTER L'ANAPHYLAXIE, ET MÉTHODE CORRESPONDANTE

(30) Priority: 05.07.2019 IT 201900011055
(43) Date of publication of application: 11.05.2022
(73) Proprietor: Da Broi, Ugo, 33100 Udine (IT)
(72) Inventor: Da Broi, Ugo, 33100 Udine (IT)
(74) Representative: Petraz, Gilberto Luigi
(86) International application number: PCT/IT2020/050166
(87) International publication number: WO 2021/005629

(56) References cited:
- CA-A1- 2 300 687
- CN-A- 103 792 368
- JP-A- 2002 162 347
- US-A- 5 716 854
- US-A- 5 744 319
- US-A1- 2003 092 092
- US-A1- 2004 047 891
- US-A1- 2009 011 445
- US-B2- 10 080 841
- JULIEN BREAULT-TURCOT ET AL: "Nanostructured substrates for portable and miniature SPR biosensors", ANALYTICAL AND BIOANALYTICAL CHEMISTRY, SPRINGER, BERLIN, DE, vol. 403, no. 6, 24 April 2012 (2012-04-24), pages 1477 - 1484, XP035057323, ISSN: 1618-2650, DOI: 10.1007/S00216-012-5963-1
- QIAN-NAN LI ET AL: "SPR biosensor detection for serum tryptase in animal of anaphylactic shock", TRANSDUCER AND MICROSYSTEM TECHNOLOGIES, vol. 32, no. 9, 1 January 2013 (2013-01-01), pages 39 - 41, XP055673892, DOI: 10.13873/j.1000-97872013.09.010
- CAUGHEY ET AL: "Tryptase genetics and anaphylaxis", JOURNAL OF ALLERGY AND CLINICAL IMMUNOLOGY, ELSEVIER, AMSTERDAM, NL, vol. 117, no. 6, 1 June 2006 (2006-06-01), pages 1411 - 1414, XP005513540, ISSN: 0091-6749, DOI: 10.1016/J.JACI.2006.02.026

## Description

### FIELD OF THE INVENTION

The invention described here concerns a portable diagnostic device, and the corresponding diagnostic method, to be used in the medical or veterinary field to analyze a biological sample of a human or animal subject and diagnose a specific hypersensitivity reaction to an allergen, in particular an anaphylactic allergic reaction, and possibly to differentiate it from an anaphylactoid reaction. The present invention is also applicable in the field of necropsy, medical or veterinary investigations, or investigations of forensic pathology to determine the causes of death of a subject. The diagnostic device is pocket-sized, and is also suitable to be used by non-expert staff, outside laboratories.

### BACKGROUND OF THE INVENTION

Hypersensitivity reactions of the immune system of a human or animal subject are known, when subjected to interaction with allergenic substances, or allergens, coming from the external environment.

Possible variants of hypersensitivity reactions may include, for example, anaphylactic allergic reactions, possibly also anaphylactic shock, mediated by immunoglobulins.

Hypersensitivity reactions can also include non-specific reactions called anaphylactoids, also induced by the interaction with allergens, which, although they are not mediated by immunoglobulins, can lead to types of symptoms and consequences similar to allergic and anaphylactic reactions.

One of the characteristics that make hypersensitivity reactions particularly dangerous is the unpredictability, or at least the only partial predictability, of the event.

This unpredictability is due to the great variety and quantity, both in nature and in artificial environments, of potentially allergenic substances, such as drugs, food, dust, substances secreted by insects, for example the venom of hymenoptera such as bees, and substances secreted by plants, for example pollen.

Each subject can have its own immune response peculiar to these allergenic substances, varying in intensity and type based on the particular allergenic substance with which it comes into contact.

Furthermore, even considering the same individual and a particular allergen, the type and intensity of the immune response and possible hypersensitivity reaction can vary over time and with the age of the subject.

Moreover, some subjects may have greater risks, such as for example asthmatics, animals that often come into contact with substances of various types, people who carry out particular jobs, such as beekeepers for example, who are particularly subject to the risk of being stung by bees.

Hypersensitivity reactions typically have a very rapid development over time, which can cause harmful effects on the subject's organism, even very intense, and can even, in the worst cases, cause death.

For example, some hypersensitivity reactions can worsen even within a few minutes from the subject's interaction with the allergenic substance, or in any case from the appearance of the first symptoms, causing serious cardiorespiratory effects and even the death of the subject affected.

Furthermore, if the hypersensitivity reaction occurs outside the hospital, the consequences can be more serious, due to the remoteness of the subject from health facilities and/or the possible lack of personnel able to provide assistance.

It is therefore necessary to diagnose the onset of hypersensitivity reactions promptly, by means of etiological and differential diagnosis, in order to be able to provide the necessary rapid emergency intervention.

In particular, with the onset of the hypersensitivity reaction, it is necessary to provide emergency and/or post-acute therapies, if the subject is in a protected health or hospital environment, or organize transport to a protected health or hospital environment, if the subject is outside the hospital.

However, the identification of hypersensitivity reactions is often problematic, since the initial symptoms can be confused with symptoms of other diseases.

Furthermore, the existing diagnostic methods for identifying possible allergic/anaphylactic reactions require too long a time to be able to intervene effectively on the subject in suitable time periods.

For example, some known methods, which could potentially be used for the diagnosis of hypersensitivity reactions, are based on enzyme-linked immunosorbent assay methods (Enzyme-Linked Immunosorbent Assay, ELISA).

However, these methods require prohibitively long analysis times with respect to the specific reaction times of hypersensitivity reactions, and cannot be used to provide a diagnosis that is timely enough to allow effective intervention on the subject.

Furthermore, these methods require laboratory devices and equipment that are often too complex and cumbersome to be used in the event of an emergency and/or in open environments, possibly outside the hospital, or far from health or hospital facilities.

The document "SPR biosensor detection for serum tryptase in animal of anaphylactic shock" from Qian-Nan Li et al. in Transducer and Microsystem Technologies (2013), pages 39-41, describes the use of a Surface Plasmon Resonance (SPR) technology intended to identify tryptase levels in laboratory animals that died from anaphylactic shock induced by the injection of human serum. This solution provides to use a mixture of antibodies (anti alpha-tryptase and anti beta-tryptase) and therefore long detection times, not compatible with the need to identify the onset of an allergic reaction in living subjects quickly. Moreover, the standard SPR technology that is described provides that the sample to be analyzed is taken into contact with the recognition surface by means of "micro flow-cell" technology, that is, with the use of continuous flow capillary channels which irrigate the analytical cell, thus requiring an auxiliary injecting device. This solution, therefore, is not suitable to be used outside a laboratory.

Document CN-A-103 792 368 describes a method to prepare the recognition surface of an optical biosensor by applying a substrate consisting of two resins, on which a muscle antigenic protein called shrimp myosin can be attached, and anti-shrimp myosin antibodies. This solution also provides to use continuous flow capillary channels to irrigate an analytical cell so that the blood sample can interact with the recognition surface. This solution, therefore, is not suitable to be used outside a laboratory.

Document US-B-10 080 841 describes a device intended to monitor respiratory function, that is, to diagnose an asthmatic attack, and the corresponding pharmacological treatment.

Document US-A-5 716 854 describes an electrochemical procedure which, by using different types of buffer solutions, modifies the pH and the environmental ionic concentration, defined as "ionic strength", in the recognition substrates of the optical biosensors or of the apparatuses for ELISA type assays (Enzyme Linked Immunosorbent Assay).

JP-A-2002 162347 describes a construction variant of a standard structure of an analyzer for optical biosensor devices.

The publication "tryptase genetics and anaphylaxis" from Caughey, G. , in J. Allergy Clin. Immunol. 2006 June, 117(6): 1411-1414, discloses using beta-tryptase as a marker in anaphylaxis.

The publication "Nanostructured substrates for portable and miniature SPR biosensors" in Anal. Bioanal. Chem. 2021 June, 403(6): 1477-1484, discloses miniaturized SPR sensors with a nanostructured metal surface for immobilizing bioreceptors thereon. The SPR signal intensity is increased in these sensors by positioning the bioreceptors in regions of the metal surface with highest electromagnetic field.

Some of the devices and methods described in these documents require specialized personnel for their application, and instruments present inside laboratories, and are not suitable to be used by unskilled people.

There is therefore a need to perfect a diagnostic method and a diagnostic device which can overcome at least one of the disadvantages of the state of the art.

One purpose of the present invention is to provide a diagnostic method and device that allow the rapid and timely identification of an allergic, anaphylactic or anaphylactoid reaction.

Another purpose of the present invention is to provide a diagnostic method and device that allow the certain identification of the hypersensitivity reaction very quickly.

Another purpose of the present invention is to provide a diagnostic method and device that allow the certain identification of the reaction even in places that are far from hospital facilities.

Another purpose of the present invention is to provide a diagnostic method and device that allow to intervene promptly when such reactions occur, even if the affected person is debilitated and/or unable to move.

Another purpose of the present invention is to provide a device which can be easily transported by a person, and which can be used by anyone, that is, both by specialized personnel and also by people without any experience whatsoever in the medical field.

The Applicant has devised, tested and embodied the present invention to overcome the shortcomings of the state of the art and to obtain these and other purposes and advantages.

### SUMMARY OF THE INVENTION

The present invention is set forth and characterized in the independent claims. The dependent claims describe other characteristics of the present invention or variants to the main inventive idea.

In accordance with the above purposes, embodiments described here concern a portable diagnostic device and the corresponding diagnostic method to analyze a biological sample of a human or animal subject to diagnose a hypersensitivity reaction in said subject, which overcome the limits of the state of the art and eliminates the defects present therein.

With the term "portable", here and hereafter in the description we mean a device that has a small format, which has small sizes and is substantially pocket-sized and not simply mobile/transportable.

The portable diagnostic device comprises a plasmonic optical biosensor comprising a bio-recognition substrate which has a recognition surface, able to receive the biological sample, and a dielectric interface surface, opposite the recognition surface.

Biochemical binders are immobilized on the bio-recognition surface, configured to selectively bind to biochemical markers associated with the hypersensitivity reaction and present in the biological sample, forming marker-binder complexes in a quantity correlated to a hypersensitivity reaction in the subject.

The plasmonic optical biosensor also comprises a plasmonic optical transducer, coupled with the bio-recognition substrate and which comprises a metal layer having an interface metal surface disposed in contact with the dielectric interface surface, defining with it a plasmonic optical interface region.

The plasmonic optical biosensor also comprises a source of electromagnetic radiation able to emit an incident radiation toward the plasmonic optical interface region.

The plasmonic optical biosensor also comprises an electromagnetic radiation detector able to detect an optical signal reflected by the plasmonic optical interface region, correlated to a quantity of marker-binder complexes which form on the recognition surface of the bio-recognition substrate, and transform it into an electrical signal.

According to the present invention, the recognition surface has a surface finish comprising a plurality of channels with micrometric and/or nanometric sizes, which increase its total area.

This solution allows to achieve the following advantages:
- possibility of placing a greater number of biochemical binders on the recognition surface;
- increase in the quantity and speed of the formation of marker-binder complexes in the unit of time;
- introducing the sample to be analyzed directly on the recognition surface, without needing to use continuous flow capillary channels, as provided in the solutions of the state of the art;
- making the device faster in the diagnostic response.

These characteristics allow to detect the occurrence of a possible allergic reaction particularly quickly, and therefore to promptly intervene to mitigate or reduce its effects.

Advantageously, the device is configured to provide a single electromagnetic signal, which can have a first reference value if the absence of a marker captured on the recognition surface is detected, and/or at least a second value, different from the first, if the presence of a biochemical marker is detected, preventing the production of a complex electromagnetic signal, as generally occurs in the case of Surface Plasmon Resonance sensors.

This avoids having to provide a group of control patients for setting the device, but only a basic setting for detecting the value of the reference signal.

Simplifying the electromagnetic signal and therefore the result provided to the user simplifies the use of the device according to the invention, making it suitable for use also by non-expert personnel.

The pocket size and the speed of response of the portable pocket-sized device according to the invention make it suitable for use by beekeepers to rapidly identify the onset of an allergic reaction while they are outside, in the vicinity of the hives that are normally placed far from inhabited centers. Furthermore, the portable device can be advantageously used by the staff of emergency medical services, ambulances, first aid services, health/hospital facilities to rapidly recognize or exclude an allergic reaction while formulating a diagnosis for a patient.

Some embodiments of the diagnostic method comprise:
- providing the portable diagnostic device described here;
- positioning a biological sample in contact with the recognition surface;
- performing an acquisition of a sample signal when the biological sample is in contact with the recognition surface;
- processing the sample signal, by comparison with a reference signal, in order to diagnose the hypersensitivity reaction;
- signaling the result of the diagnosis.

According to some embodiments, the method provides to use, as a reference signal, a signal acquired in the absence of a biological sample

According to some embodiments, the biological sample is positioned in a positioning portion, and the acquisition of the sample signal and of the reference signal provides to insulate and close the positioning portion, in order to prevent disturbances due to radiation, magnetic fields, air, water and humidity, coming from the environment outside the positioning portion.

In particular, the positioning portion can be insulated from possible optical and/or electromagnetic radiations or possible magnetic fields that could interfere with the acquisition of the signals.

Advantageously, the portable diagnostic device and the diagnostic method can be used to provide a safe diagnosis of a hypersensitivity reaction, since it is possible to identify specific biochemical markers associated with such reaction, in the rapid times typical of plasmonic spectroscopy, even away from hospital facilities, since all the components are integrated in a single portable device.

Other embodiments concern a portable diagnostic kit comprising at least one portable diagnostic device according to the invention and an injector device, configured to administer a therapeutic dose of drug to the subject.

### DESCRIPTION OF THE DRAWINGS

These and other aspects, characteristics and advantages of the present invention will become apparent from the following description of some embodiments, given as a non-restrictive example with reference to the attached drawings wherein:
- figs. 1 and 2 are schematic representations of a portable diagnostic device in accordance with some embodiments described here;
- fig. 3 is a schematic representation of a plasmonic optical biosensor comprised in a portable diagnostic device in accordance with some embodiments described here;
- fig. 4 and 5 schematically show some steps of a diagnostic method in accordance with some embodiments described here.
- fig. 6 is a schematic representation of a plasmonic optical biosensor comprised in a portable diagnostic device in accordance with some variants;
- fig. 7 is a schematic representation of a plasmonic optical biosensor comprised in a portable diagnostic device in accordance with some variants.

To facilitate comprehension, the same reference numbers have been used, where possible, to identify identical common elements in the drawings. It is understood that elements and characteristics of one embodiment can conveniently be incorporated into other embodiments without further clarifications.

### DESCRIPTION OF SOME EMBODIMENTS

We will now refer in detail to the various embodiments of the invention, of which one or more examples are shown in the attached drawings. Each example is supplied by way of illustration of the invention and shall not be understood as a limitation thereof. For example, the characteristics shown or described insomuch as they are part of one embodiment can be adopted on, or in association with, other embodiments to produce another embodiment. It is understood that the present invention shall include all such modifications and variants.

Before describing these embodiments, we must also clarify that the present description is not limited in its application to details of the construction and disposition of the components as described in the following description using the attached drawings. The present description can provide other embodiments and can be obtained or executed in various other ways. We must also clarify that the phraseology and terminology used here is for the purposes of description only, and cannot be considered as limitative.

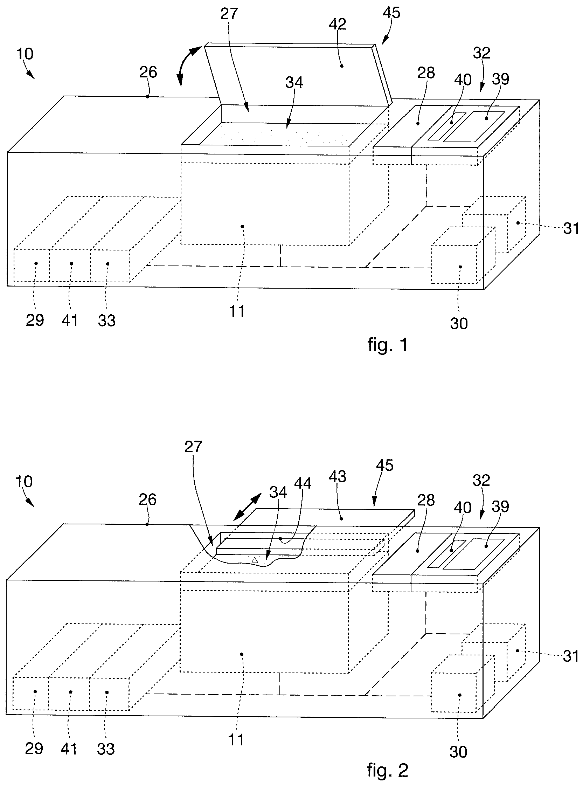

The present invention concerns a portable diagnostic device 10, schematically described by means of figs. 1, 2 and 3, which can be used to analyze a biological sample of a human or animal subject to diagnose a hypersensitivity reaction in said subject.

Hypersensitivity reactions refer to all those types of immune responses of the organism induced by the subject's exposure to allergenic substances that come from the external environment.

Hypersensitivity reactions can comprise allergic reactions, anaphylactic reactions, anaphylactic shocks, anaphylactoid reactions.

The device 10 can be used by an operator, who can him/herself possibly be the subject, if human, and if in suitable physical condition, or a third person, if the subject is an animal, or if the subject is human but he/she is not in suitable physical condition.

The biological sample can be capillary blood or venous blood or arterial blood or other biological material, previously taken from the subject, suitable to be analyzed by means of the device 10.

The device 10 comprises a plasmonic optical biosensor 11, able to identify and detect the presence of one or more biochemical markers 12 present in the biological sample.

The biochemical markers 12, associated with the hypersensitivity reaction, are present in the biological sample when the hypersensitivity reaction is in progress.

The identification of the biochemical markers 12 allows to associate the symptoms of the illness felt by the subject at the onset of the hypersensitivity reaction, excluding other possible causes, for example diseases or other syndromes, at the origin of these symptoms (differential diagnosis).

In particular, the identification of the biochemical markers 12 allows to directly diagnose anaphylactic hypersensitivity reactions associated with them.

If the biochemical markers 12 associated with anaphylactic hypersensitivity reactions are not identified, it is possible to diagnose, by exclusion, the anaphylactoid hypersensitivity reactions when the subject presents symptoms which can be associated with them.

The biochemical markers 12 can be any molecule, macromolecule, supramolecular or cellular structure, produced and/or released by the human or animal organism following interaction with an allergen, following a chain of biochemical processes triggered by contact of the organism with the complex formed by the allergen and the antibodies produced by the organism itself.

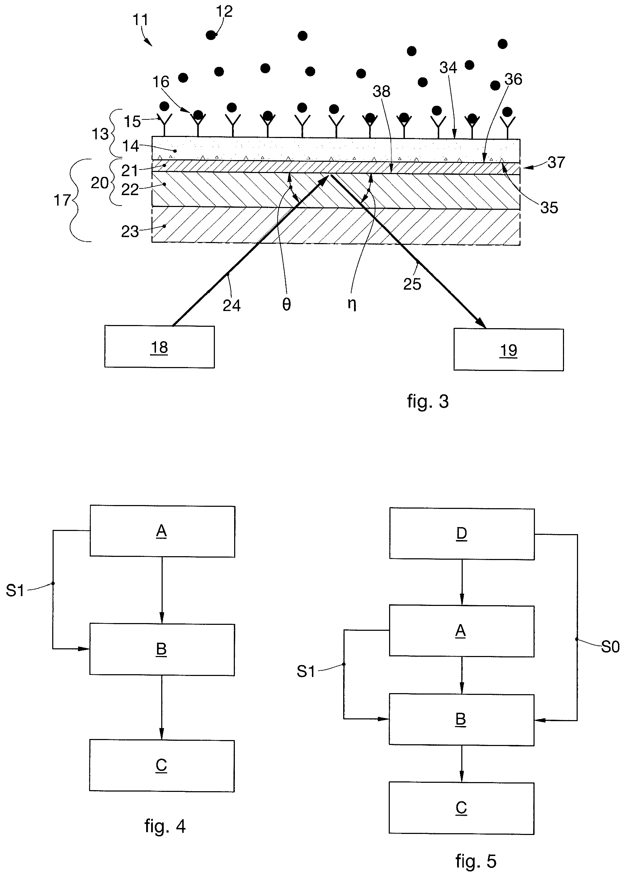

In accordance with the invention schematically described with reference to fig. 3, the plasmonic optical biosensor 11 comprises a bio-recognition substrate 13, configured to recognize and chemically bind the biochemical markers 12 present in the biological sample of the subject.

The bio-recognition substrate 13 can exhibit optical properties of the dielectric type.

The bio-recognition substrate 13 has a recognition surface 34, able to receive the biological sample, and a dielectric interface surface 35, opposite the recognition surface 34.

In particular, biochemical binders 15 are immobilized on the recognition surface 34, configured to selectively bind to the biochemical markers 12 associated with the hypersensitivity reaction present in the biological sample.

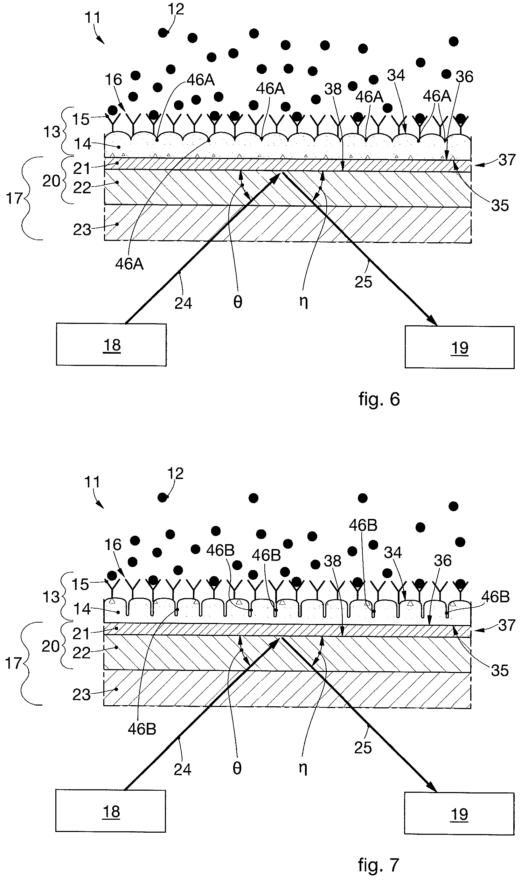

According to the present invention, as for example represented with reference to figs. 6 and 7, the recognition surface 34 has a surface finish comprising a plurality of channels 46 that have micrometric and/or nanometric sizes. In other words, the recognition surface 34 is not uniform but is modeled with a plurality of micro-nano channels 46 in order to increase its total area.

In this way, it is possible to position a large number of biochemical binders 15 on the recognition surface, even over 100% more than a smooth and uniform surface, thus increasing the quantity and speed of formation of marker-binder complexes, and consequently increasing the analysis speed.

In particular, the number of biochemical binders 15 can be comprised between 100% and 200%, or possibly higher, in relation to the number and depth of the micro-nano channels 46 with respect to the quantity that can be positioned on a smooth and uniform recognition surface 23, which doesn't have this surface finish.

According to some embodiments, the micro-nano channels 46 can be made as hollows 46A (fig. 6) made between rounded portions, or as grooves 46B that have a desired depth (fig. 7), made in the thickness of the surface portion of the bio-recognition substrate 13.

In embodiments which do not fall under the scope of the claims, the biochemical binders 15 can be previously selected on the basis of the type of hypersensitivity reaction to be diagnosed, or also on the basis of the characteristics of the subject's immune system, thus providing the possibility of using the present invention for a wide range of hypersensitivity reactions and for a wide range of subjects.

The biochemical markers 12 and the biochemical binders 15 form marker-binder complexes 16, in a quantity correlated to the hypersensitivity reaction in the subject.

In some embodiments, the bio-recognition substrate 13 can comprise a solid phase 14 of dextran, on which the biochemical binders 15 are immobilized. The biochemical binders are only anti-beta-tryptase antibodies. The biochemical markers 12 can comprise beta-triptase and the biochemical binders 15 are specific monoclonal or polyclonal antibodies selective toward beta-triptase. Providing specific antibodies that bind only to beta-triptase allows to increase the speed of the analytical procedure, as well as ensuring greater accuracy and sensitivity with respect to those that can be obtained with mixtures of anti α-triptase and anti β-triptase antibodies.

The marker-binder complexes 16 can be immunocomplexes of the antigen-antibody type.

The plasmonic optical biosensor 11 also comprises a plasmonic optical transducer 17 coupled to the bio-recognition substrate 13 and having a metal layer 21 having an interface metal surface (36) disposed in contact with said dielectric interface surface (35) defining a plasmonic optical interface region (37).

The plasmonic optical transducer 17 is suitable to detect the formation of the marker-binder complexes 16 and provide an optical signal 25.

The plasmonic optical transducer 17 can detect the formation of the marker-binder complexes 16 by means of spectroscopic techniques, in particular based on surface plasmon resonance (SPR).

In some embodiments, the plasmonic optical transducer 17 comprises a sensor chip 20, sensitive to variations in the optical properties of the bio-recognition substrate 13.

In the present description, optical properties are understood as the properties of the materials that characterize the light-matter interaction, such as for example the refractive index, relative permittivity, electrical susceptibility, dielectric function, which can be understood as real and/or complex functions that depend on the frequency of an incident radiation 24.

In some embodiments, the sensor chip 20 comprises the metal layer 21 and a dielectric substrate 22, made of materials which have optical properties suitable to generate surface plasmonic resonances.

In some embodiments, the dielectric substrate 22 can be made of a vitreous material and have optical properties of the dielectric type.

In some embodiments, the dielectric substrate 22 can have a millimeter thickness. For example, the thickness of the dielectric substrate 22 can be variable between 1 and 10mm, as a function of applications.

In some embodiments, the metal layer 21 can be made of metallic material, advantageously gold or silver, and have optical properties of the metallic type.

In some embodiments, the metal layer 21 can have a variable thickness, from a few nanometers to a few tens of nanometers.

According to some embodiments, the metal layer 21 has a first interface metal surface 36 disposed in contact with the dielectric interface surface 35, and a second surface 38, opposite the first surface and disposed in contact with the dielectric substrate 22.

A plasmonic optical interface region 37 is thus defined, corresponding in particular to the region of the metal layer 21 which is in the proximity of the plane on which the contact between the dielectric interface surface 35 and the interface metal surface 36 occurs.

In this region, the optical properties of the metal layer 21 are influenced by the optical properties of the bio-recognition substrate 13.

The variations of the optical properties of the bio-recognition layer 13, caused by the formation of the marker-binder complexes 16, therefore induce variations of the optical properties of the metal layer 21. These variations can be of a relatively small order of magnitude, but are nevertheless sufficient to modify the light-matter interaction characteristics of the sensor chip 20 and therefore the characteristics of the optical signal 25 emitted.

The plasmonic optical biosensor 11 also comprises a source of electromagnetic radiation 18, able to emit the incident radiation 24 toward the plasmonic optical interface region 37.

The incident radiation 24 has physical characteristics, for example frequency, intensity, phase, and geometric characteristics, for example an angle of incidence θ with respect to the plane of contact between the dielectric interface surface 35 and the interface metal surface 36, suitable to generate some surface plasmonic resonances in the plasmonic optical interface region 37.

In some embodiments, the source of electromagnetic radiation 18 can emit the incident radiation 24 with a frequency associated with the infrared and/or visible spectrum.

In some embodiments, the source of electromagnetic radiation 18 can emit the incident radiation 24 with a frequency associated with the plasma frequency of the metal layer 21.

In some embodiments, the incident radiation 24 can be monochromatic light and the source of electromagnetic radiation 18 can comprise a monochromator.

In some embodiments, the incident radiation 24 can be polarized monochromatic light and the source of electromagnetic radiation 18 can comprise a monochromator and a polarizer.

The physical and geometric properties of the incident radiation 24 are modified by the interaction with the sensor chip 20, in particular inducing the surface plasmon resonances in the plasmonic optical interface region 37, and the extent of such modification depends on the optical properties of the sensor chip 20.

A reflected radiation, that is, an optical signal 25, is then emitted with an angle of reflection η, possibly different from the angle of incidence θ, which has physical and geometric properties possibly different from those of the incident radiation 24.

The difference between the physical and geometric properties of the optical signal 25 with respect to those of the incident radiation 24 is correlated to the variation of the optical properties of the sensor chip 20, in turn correlated to the formation of the marker-binder complexes 16.

The optical signal 25 is therefore correlated to the quantity of marker-binder complexes 16 which are formed on the recognition surface 34 of the bio-recognition substrate 13.

The plasmonic optical biosensor 11 comprises an electromagnetic radiation detector 19 able to detect the optical signal 25 reflected by the plasmonic optical interface region 37 and transform it into an electrical signal, readable by other electronic and/or analog and/or digital devices or components.

In some embodiments, the electromagnetic radiation detector 19 can measure the frequency and/or the angle of reflection η and/or the intensity of the optical signal 25.

When the source of electromagnetic radiation 18 is activated, the incident radiation 24 passes through the dielectric substrate 22 and interacts with the metal layer 21, generating plasmonic resonances, to which corresponds a certain optical signal 25 emitted.

If the biological sample is absent, that is, it is not positioned on the recognition surface 34, the optical signal 25 detected corresponds to a reference signal S0.

If the biological sample is present, that is, it is positioned on the recognition surface 34, the optical signal 25 detected is correlated to the characteristics of the biological sample, that is, it corresponds to a sample signal S1.

According to some embodiments, the device 10 is configured to supply a single electromagnetic signal, which can be the negative reference signal S0 if the absence of a biochemical marker 12 captured on the recognition surface 34 is detected, and/or a sample signal S1, if the presence of a marker 12 is detected.

In some embodiments, the plasmonic optical transducer 17 can also comprise a prismatic structure 23, interposed between the source of electromagnetic radiation 18 and the dielectric substrate 22, which, when hit by the incident radiation 24, generates an evanescent field, which interacts with the sensor chip 20.

In such embodiments, the dielectric substrate 22 can adhere to the prismatic structure 23, so as to prevent dispersion of the evanescent field.

These embodiments have the advantage of providing more uniform and reproducible optical signals 25, since the sensor chip 20 is irradiated by the evanescent field, which is more uniform than the incident radiation 24.

In embodiments schematically described with reference to figs. 1 and 2, the device 10 also comprises a containing body 26, in which the biosensor 11 is integrated, which defines the volume and bulk of the device 10 itself.

In some embodiments, the containing body 26 can be configured to protect the device 10 from environmental and meteorological factors, such as, for example, water, rain, humidity, significantly high and/or low temperatures, high solar radiation, and also from electrical discharges and short circuits deriving from the contact between humidity and the electrical components inside the device 10.

For this purpose, the containing body 26 can be made of resistant and nonconductive material, for example plastic or rubber.

In some embodiments, the containing body 26 can be configured to protect the device 10 from mechanical stresses, such as for example falls, shocks, impacts, or other. These characteristics make the device 10 more resistant and therefore suitable to be moved even abruptly without it breaking or being damaged.

Such characteristics allow to use the device 10 both in closed environments, for example inside a hospital facility, and also in open environments.

Advantageously, such characteristics make the device 10 suitable to be used, for example, by beekeepers subjected to the risk of being stung by hymenoptera.

In some embodiments, the device 10 has volume, bulk and weight specifications limited to the point of allowing easy transport by hand.

The device 10 can have a suitable volume, bulk and weight to be placed in a pocket of an item of clothing, or in a pocket of a backpack, or in a walking bag.

In some embodiments, the device 10 can be configured in the shape substantially of a parallelepiped, the longer side of which has a length comprised between 5 and 15cm.

In some embodiments, the device 10 can have sizes and shapes similar to a common pack of cigarettes.

In some embodiments, the device 10 can have a weight comprised between 50 and 250 grams, in particular between 100 and 200 grams.

In some embodiments, the device 10 comprises a positioning portion 27 suitable to house a predetermined quantity, advantageously small, of the biological sample of the subject.

The positioning portion 27 can be configured as a cavity, made inside the containing body 26, below which the plasmonic optical biosensor 11 can be positioned.

In such embodiments, the recognition surface 34 of the plasmonic optical biosensor 11 can be configured, at least partly, as the bottom of the positioning portion 27.

According to some embodiments, the device 10 comprises covering means 45 configured to selectively close and insulate the positioning portion 27 at least during a step of acquiring and/or processing a biological sample.

The covering means 45 can be configured to electromagnetically insulate the positioning portion 27, in particular from electromagnetic radiation and magnetic fields coming from the external environment.

The covering means 45 can be configured to hermetically close the positioning portion 27, so as to make it impermeable to liquids and gaseous substances coming from the external environment.

The covering means 45 can be made of opaque material, in particular optically and electromagnetically insulating, and impermeable to liquids and gaseous substances, for example water and air, coming from the external environment.

These characteristics allow to prevent the solid phase 14 and/or the biochemical binders 15 from degrading upon contact with external light and/or with the water and air coming from the external environment, and the electrical components of the device 10 from being damaged or corroded by humidity.

Furthermore, as will become clearer hereafter, these characteristics mean that during the step of acquiring the signals S0, S1, the only electromagnetic radiation that interacts with the biological sample is the incident radiation 24, without other disturbances due to other external sources of radiation.

These characteristics mean that during the step of acquiring the signals S0, S1, the positioning portion 27 is insulated from external magnetic fields, which could interact with the metal layer 21, undesirably modifying the plasmonic resonances.

In particular, in embodiments described by way of example by means of fig. 1, the positioning portion 27 can be made open toward an upper surface of the containing body 26, and the covering means 45 can be configured as a mobile door 42.

In other embodiments, described by way of example by means of fig. 2, the positioning portion 27 can be made inside the containing body 26, and be accessible through an insertion aperture 44 in correspondence with a lateral surface.

According to this embodiment, insertion means 43 can be provided, for example a plate on which the biological sample can be positioned, which can be inserted in the positioning portion 27 through the insertion aperture 44. According to this embodiment, the covering means 45 can be defined by the portions of the containing body 26 that delimit the positioning portion 27.

Furthermore, the insertion means 43 can be configured to close the positioning portion 27 hermetically and so that it is electromagnetically insulated in correspondence with the aperture 44, themselves acting as covering means 45.

It is obvious to the person of skill in the art that the embodiments described so far by means of fig. 1 and fig. 2 can be combined with each other, and that the device 10 can comprise both the mobile door 42 and also the insertion means 43.

In some embodiments, the device 10 of the present invention also comprises processing means 33, which can for example comprise a processor (CPU) and a software, installed in a memory unit present in the device 10, which can manage the functioning of the device 10 and the user interface with the operator.

In some embodiments, the processing means 33 are configured to process the electrical signal transmitted by the electromagnetic radiation detector 19, and determine whether the quantity of the recognition complexes 16 detected falls within a quantity range indicative of a hypersensitivity reaction in the subject.

In some embodiments, the processing means 33 can provide a positive or negative presence diagnosis, which respectively indicates the presence or absence of a hypersensitivity reaction in the subject.

In some embodiments, the processing means 33 can provide a severity diagnosis, which indicates the level of severity of the hypersensitivity reaction in the subject, for example classifying it as "mild", "moderate", "severe", "very severe", on the basis of the quantity of marker-binder complexes 16 detected.

According to some embodiments, the device 10 comprises signaling means 32 configured to provide the operator with information correlated to the presence and/or intensity of a hypersensitivity reaction detected in the biological sample.

The processing means 33 can therefore signal the diagnosis to the operator by means of the signaling means 32.

In some embodiments, the signaling means 32 can comprise LEDs which light up with different colors, with predetermined color codes, based on a positive or negative outcome of the presence diagnosis, or on the basis of the severity level of the severity diagnosis.

In some embodiments, useful for example in cases where the subject is visually impaired or has difficulty in distinguishing colors, the signaling means 32 can comprise acoustic warning devices, which communicate the diagnosis.

In some embodiments, the signaling means 32 can comprise a screen 39, which can graphically display the data processed by the processing means 33 and/or the result of the diagnosis.

In some embodiments, it is possible to graphically display on the screen 39 information correlated to the quantity of marker-binder complexes 16 that form on the recognition surface 34 of the bio-recognition substrate 13.

According to some embodiments, the screen 39 is configured to display an indication of the presence or absence of an allergic reaction, and/or its intensity.

For example, it is possible to display "+" or "-" signs, or a plurality of lines to identify a positive diagnosis, possibly with a number of lines correlated to the intensity of reaction detected, graphs with different colors based on the level of intensity, or a sentence, or more.

According to other embodiments, the signaling means 32 can comprise an acoustic signaler 40, suitable to generate an acoustic signal, for example a succession of beeps, or sounds, or a vocal signal, to indicate the diagnosis processed.

In some embodiments, the device 10 can have control means 28, for example buttons, levers, keypads, a touch screen, suitable to allow the operator to interact with the device 10, for example turning it on/off, or activating the source of electromagnetic radiation 18 and the acquisition of the signals S0, S1.

According to some embodiments, the control means 28 and the signaling means 32 can be integrated in a single component, for example in the case of a touch screen 39.

The device 10 can also comprise power supply means 29, for example batteries, suitable to supply electrical power to all the components of the device 10.

According to some embodiments, the batteries can be of the disposable or rechargeable type. Particularly in the latter case, the signaling means 32 can also be configured to provide indications on the state of charge of the batteries.

The device 10 can also comprise localization means 30 configured to detect the geo-localization coordinates of the device 10.

In some embodiments, the localization means 30 can use a digital map stored in the device 10 and/or can use a digital map available on the internet, by means of a connection to a cellular or internet network, provided by suitable communication means 31 comprised in the device 10 .

The communication means 31 can be configured to provide a connection to a cellular or internet network.

In some embodiments, the communication means 31 can send an emergency communication by telephone or internet network to a previously determined recipient.

In some embodiments, the communication means 31 can be configured to perform from simple data communications, for example sending an emergency signal and/or geo-localization coordinates of the device 10, up to voice communications.

In some embodiments, the communication means 31 can be configured to provide a wireless, Bluetooth or other type of connection with a mobile communication device, for example a mobile phone, with the possibility of exploiting the means provided in the latter to carry out the emergency communication.

Some embodiments also concern a diagnostic method to analyze a biological sample of a human or animal subject to diagnose a hypersensitivity reaction in said subject.

In some embodiments, the method of the present invention provides to:
- provide the portable diagnostic device 10 described here;
- position a biological sample in contact with a recognition surface 34;
- perform an acquisition A of a sample signal S1 on the biological sample placed in contact with the recognition surface 34;
- process B the sample signal S1, by comparison with a reference signal S0 to diagnose the hypersensitivity reaction;
- signal C the result of the diagnosis.

For example, a drop of the biological sample, in particular blood, can be positioned directly on the recognition surface 34, on which it can remain stationary in a static position during the acquisition of the sample signal S1.

Fig. 4 schematically shows the execution of the acquisition A, processing B and signaling C.

In some embodiments, the reference signal S0 can be previously acquired and stored in the device 10.

According to some variants, the reference signal S0 can be a signal derived from experimental tables or results, and be stored in the device 10 during the production step.

In alternative embodiments, it is provided to obtain the reference signal S0 during the use of the device 10, performing an acquisition of a reference D, in the absence of the biological sample.

According to some embodiments, the method can provide to perform the acquisition of a reference D only once, for example the first time the device 10 is turned on, and store it in the memory unit 41 so as to have it available when necessary. This can be useful, for example, if the device 10 is used by a single subject.

According to some variants, it can be provided that the acquisition of a reference D is performed each time the device 10 is used, before the processing step B.

For example, in embodiments described with reference to fig. 5, the acquisition of a reference D can be performed before the acquisition of the sample A.

The biological sample can be positioned in the positioning portion 27, in contact with the recognition surface 34.

The acquisitions A, D can provide to electromagnetically insulate and hermetically seal the positioning portion 27, in order to prevent disturbances due to radiation, magnetic fields, air, water and humidity, coming from the external environment to the positioning portion 27.

In some embodiments, the positioning can occur by directly positioning the biological sample on the positioning portion 27.

In some embodiments, the positioning can occur by adsorbing the biological sample on a strip of disposable adsorbent material, and positioning the strip on the positioning portion 27.

In the event the positioning portion 27 is made substantially closed, as shown in fig. 2, the strip of disposable adsorbent material can be inserted into the device 10 by means of the insertion means 43.

These embodiments are particularly advantageous in cases where the biological sample consists of liquid or fluid substances, such as blood.

Performing the acquisition of the sample A and/or the acquisition of a reference D provides that the operator activates, by means of the control means 28, the emission of the incident radiation 24 by the source of electromagnetic radiation 18.

The electromagnetic radiation detector 19 then transforms, according to known modes, the optical signal 25 into the electrical signal and sends it to the processing means 33.

The processing B of the sample S1 and reference S0 signals provides to compare the reference signal S0 with the sample signal S1, in order to detect the difference in physical and geometric properties between the incident radiation 24 and the optical signal 25, caused by the formation of marker-binder complexes 16.

In some embodiments, the processing B of such signals S0, S1 can provide to calculate at least one ratio between the sample signal S1 and the reference signal S0, in particular chosen from:
- the ratio between the intensity of the sample signal S1, in particular of the optical signal 25 associated with the sample signal S1, and the intensity of the reference signal S0, in particular of the optical signal 25 associated with the reference signal S0;
- the ratio between the frequency shift of the sample signal S1 and the frequency shift of the reference signal S0, wherein frequency shift is understood as the difference in frequency between the optical signal 25 and the incident radiation 24 associated with the respective signals S0, S1;
- the ratio between the phase variation of the sample signal S1 and the phase variation of the reference signal S0, wherein phase variation is understood as the difference between the angle of incidence θ of the incident radiation 24 and the angle of reflection η of the optical signal 25, associated with the respective signals S0, S1.

For example, if the ratio between the signals S0 and S1 is about 1, the processing means 33 process a negative presence diagnosis, associated with the absence of a hypersensitivity reaction in the subject.

If this ratio is lower/higher than 1 by a given threshold value, the processing means 33 process a positive presence diagnosis, associated with the presence of a hypersensitivity reaction in the subject.

According to other embodiments, a plurality of threshold values can be stored in the memory unit 41, each defining a determinate level of intensity of the allergic reaction, and the processing means 33 can provide a severity diagnosis by comparing the ratio S0/S1 with the stored values, so as to identify a "mild", "moderate", "serious", "very serious" hypersensitivity reaction.

In some embodiments, the signaling C provides that the processing means 33 signal, through the signaling means 32, the outcome of the diagnosis, according to the modes previously described.

According to possible embodiments, the signaling C provides that the type and/or quantity of drugs to be administered to the subject for an effective therapeutic treatment of the ongoing hypersensitivity reaction is displayed on the screen 39, based on the quantity of marker-binder complexes 16 detected and/or the severity level of the severity diagnosis.

In some embodiments, the signaling C provides, following a positive presence diagnosis, to activate the communication means 31 to send an emergency call to an emergency number associated with a healthcare service provider and/or a private number previously stored in the device 10.

In some embodiments, the signaling C provides, following a positive presence diagnosis, to activate the localization means 30 in order to detect the geo-localization coordinates of the device 10.

These coordinates can be transmitted automatically during the emergency call by the communication means 31.

In the embodiments in which the communication means 31 are configured to interface with a mobile communication device, for example a mobile phone, in addition to or as an alternative to signaling C the diagnosis by means of signaling means 32, it is also possible that the signaling C is performed by means associated with the mobile device, for example on the screen, with the ringtone, or vibration.

Some embodiments also concern a portable diagnostic kit to analyze a biological sample of a human or animal subject to diagnose a hypersensitivity reaction in said subject and to administer, if necessary, a therapeutic dose of a drug, for example containing adrenaline, to said subject.

The diagnostic kit of the present invention comprises at least the portable diagnostic device 10 described here and an injector device, configured to administer a therapeutic dose of drug, in particular adrenaline, to a subject in whom a hypersensitivity reaction has been diagnosed.

In some embodiments, the doses delivered by the injector device can be standard doses, or doses previously calibrated on the basis of the weight of the subject that uses the diagnostic kit.

In some embodiments, the diagnostic device 10 and the injector device can be interfaced with each other, in particular the diagnostic device 10 can control the drive of the administration of the therapeutic dose of adrenaline.

It is clear that, although the present invention has been described with reference to some specific examples, a person of skill in the art shall certainly be able to achieve many other equivalent forms of device 10 and method, having the characteristics as set forth in the claims and hence all coming within the field of protection defined thereby.

In the following claims, the sole purpose of the references in brackets is to facilitate reading: they must not be considered as restrictive factors with regard to the field of protection claimed in the specific claims.

## Claims

1. Portable pocket-sized diagnostic device to analyze a biological sample of a human or animal subject to diagnose a hypersensitivity reaction in said subject, said device comprising a plasmonic optical biosensor comprising:
i) a bio-recognition substrate (13) which has a recognition surface (34), able to receive said biological sample and on which biochemical binders (15) are immobilized, configured to selectively bind to biochemical markers (12) associated with said hypersensitivity reaction present in said biological sample, forming marker-binder complexes (16) in a quantity correlated to a hypersensitivity reaction in said subject, and a dielectric interface surface (35), opposite said recognition surface (34), wherein said recognition surface (34) has a surface finish comprising a plurality of channels with micrometric and/or nanometric sizes and said biochemical binders (15) are only anti beta-tryptase antibodies;
ii) a plasmonic optical transducer (17) coupled with said bio-recognition substrate (13) and comprising a metal layer (21) having an interface metal surface (36) disposed in contact with said dielectric interface surface (35) defining a plasmonic optical interface region (37);
iii) a source of electromagnetic radiation (18) able to emit an incident radiation (24) toward said plasmonic optical interface region (37);
iv) an electromagnetic radiation detector (19) able to detect an optical signal (25) reflected by said plasmonic optical interface region (37), correlated to a quantity of marker-binder complexes (16) which form on the recognition surface (34) of said bio-recognition substrate (13), and transform it into an electrical signal.

2. Device as in claim 1, **characterized in that** said biochemical markers (12) comprise beta-tryptase.

3. Device as in any claim hereinbefore, **characterized in that** said bio-recognition substrate (13) comprises a solid phase (14) of dextran on which the biochemical binders (15) are immobilized.

4. Device as in any claim hereinbefore, **characterized in that** it also comprises processing means (33) configured to process said electrical signal and determine whether said quantity of said marker-binder complexes (16) falls within a quantity range indicative of a hypersensitivity reaction in said subject.

5. Device as in any claim hereinbefore, **characterized in that** it comprises signaling means (32) configured to provide an operator with information correlated to the presence and/or intensity of a hypersensitivity reaction in the biological sample correlated to the electrical signal obtained.

6. Device as in any claim hereinbefore, **characterized in that** it also comprises localization means (30) configured to detect the geo-localization coordinates of said device (10).

7. Device as in any claim hereinbefore, **characterized in that** it also comprises communication means (31) configured to send an emergency communication by telephone network or internet network to a previously determined recipient.

8. Device as in any claim hereinbefore, **characterized in that** it also comprises a containing body (26) configured to protect the device (10) from mechanical stresses and/or environmental and meteorological factors in which a positioning portion (27) is made, suitable to house a predetermined quantity of said biological sample and configured as a cavity, the bottom of which consists, at least in part, of said recognition surface (34), and covering means (45) configured to selectively close and insulate said positioning portion (27).

9. Device as in claim 8, **characterized in that** said covering means (45) are configured to electromagnetically insulate and hermetically seal said positioning portion (27).

10. Portable diagnostic kit to analyze a biological sample of a human or animal subject comprising at least one pocket-sized diagnostic device 10 as in any claim from 1 to 9, and an injector device, configured to administer to said subject a therapeutic dose of drug, in particular containing adrenaline.

11. Diagnostic method to analyze a biological sample of a human or animal subject to diagnose a hypersensitivity reaction in said subject, said method comprising:
- providing a portable pocket-sized diagnostic device (10) as in any claim from 1 to 9;
- positioning a biological sample directly on and in contact with the recognition surface (34);
- performing an acquisition of a sample signal (S1) when said biological sample is in contact with said recognition surface (34);
- processing said sample signal (S1), by comparison with a reference signal (S0) in order to diagnose said hypersensitivity reaction;
- signaling the result of said diagnosis.

12. Method as in claim 11, **characterized in that** it comprises, before said processing, performing a reference acquisition, in the absence of said biological sample, to obtain said reference signal (S0).

13. Method as in claim 11 or 12, **characterized in that** the biological sample is positioned on said recognition surface (34) inside a positioning portion (27) **and in that** the acquisition of said signals (S1, S0) takes place when the positioning portion (27) is electromagnetically insulated and hermetically sealed, in order to prevent disturbances due to radiation, magnetic fields, air, water and humidity, coming from the environment outside said positioning portion (27).

14. Method as in any claim from 11 to 13, **characterized in that** said processing provides to calculate at least a ratio between the sample signal (S1) and the reference signal (S0), chosen from:
- a ratio between the intensity of the sample signal (S1) and the intensity of said reference signal (S0);
- a ratio between the frequency shift of the sample signal (S1) and the frequency shift of the reference signal (S0);
- a ratio between the phase variation of the sample signal (S1) and the phase variation of the reference signal (S0).

15. Method as in any claim from 11 to 14, **characterized in that** said signaling comprises at least one of either sending an emergency communication by telephone network or internet network to a previously determined recipient or detecting the geo-localization coordinates of the device (10) and transmitting them to said recipient by means of said emergency communication.

## Patentansprüche

1. Tragbare Diagnosevorrichtung in Taschengröße zum Analysieren einer biologischen Probe eines menschlichen oder tierischen Subjekts, um eine Überempfindlichkeitsreaktion in dem Subjekt zu diagnostizieren, wobei die Vorrichtung einen plasmonischen optischen Biosensor umfasst, der Folgendes umfasst:
i) ein Bioerkennungssubstrat (13), das eine Erkennungsoberfläche (34) aufweist, die in der Lage ist, die biologische Probe aufzunehmen, und auf der biochemische Binder (15) immobilisiert sind, die konfiguriert sind, um selektiv an biochemische Marker (12) zu binden, die mit der in der biologischen Probe vorliegenden Überempfindlichkeitsreaktion assoziiert sind, wobei Marker-Binder-Komplexe (16) in einer Menge gebildet werden, die mit einer Überempfindlichkeitsreaktion in dem Subjekt korreliert, und eine dielektrische Grenzfläche (35) gegenüber der Erkennungsoberfläche (34), wobei die Erkennungsoberfläche (34) eine Oberflächenbeschaffenheit aufweist, die eine Vielzahl von Kanälen mit mikrometrischen und/oder nanometrischen Größen umfasst, und die biochemischen Binder (15) nur Anti-beta-Tryptase-Antikörper sind;
ii) einen plasmonischen optischen Wandler (17), der mit dem Bioerkennungssubstrat (13) gekoppelt ist und eine Metallschicht (21) umfasst, die eine Grenzflächenmetalloberfläche (36) aufweist, die in Kontakt mit der dielektrischen Grenzfläche (35) angeordnet ist, die einen plasmonischen optischen Grenzflächenbereich (37) definiert;
iii) eine Quelle elektromagnetischer Strahlung (18), die in der Lage ist, eine einfallende Strahlung (24) in Richtung des plasmonischen optischen Grenzflächenbereichs (37) zu emittieren;
iv) einen Detektor elektromagnetischer Strahlung (19), der in der Lage ist, ein optisches Signal (25) zu detektieren, das von dem plasmonischen optischen Grenzflächenbereich (37) reflektiert wird und mit einer Menge von Marker-Binder-Komplexen (16), die sich auf der Erkennungsoberfläche (34) des Bioerkennungssubstrats (13) bilden, korreliert, und es in ein elektrisches Signal zu transformieren.

2. Vorrichtung nach Anspruch 1, **dadurch gekennzeichnet, dass** die biochemischen Marker (12) beta-Tryptase umfassen.

3. Vorrichtung nach einem der vorhergehenden Ansprüche, **dadurch gekennzeichnet, dass** das Bioerkennungssubstrat (13) eine feste Phase (14) aus Dextran umfasst, auf der die biochemischen Binder (15) immobilisiert sind.

4. Vorrichtung nach einem der vorhergehenden Ansprüche, **dadurch gekennzeichnet, dass** sie auch Verarbeitungsmittel (33) umfasst, die konfiguriert sind, das elektrische Signal zu verarbeiten und zu bestimmen, ob die Menge der Marker-Binder-Komplexe (16) in einen Mengenbereich fällt, der eine Überempfindlichkeitsreaktion in dem Subjekt anzeigt.

5. Vorrichtung nach einem der vorhergehenden Ansprüche, **dadurch gekennzeichnet, dass** sie Signalisierungsmittel (32) umfasst, die konfiguriert sind, einem Bediener Informationen bereitzustellen, die mit dem Vorhandensein und/oder der Intensität einer mit dem erhaltenen elektrischen Signal korrelierten Überempfindlichkeitsreaktion in der biologischen Probe korrelieren.

6. Vorrichtung nach einem der vorhergehenden Ansprüche, **dadurch gekennzeichnet, dass** sie auch Lokalisierungsmittel (30) umfasst, die konfiguriert sind, die Geolokalisierungskoordinaten der Vorrichtung (10) zu erfassen.

7. Vorrichtung nach einem der vorhergehenden Ansprüche, **dadurch gekennzeichnet, dass** sie auch Kommunikationsmittel (31) umfasst, die konfiguriert sind, eine Notfallkommunikation über ein Telefonnetz oder ein Internetnetz an einen zuvor bestimmten Empfänger zu senden.

8. Vorrichtung nach einem der vorhergehenden Ansprüche, **dadurch gekennzeichnet, dass** sie auch einen Aufnahmekörper (26), der konfiguriert ist, die Vorrichtung (10) vor mechanischen Belastungen und/oder Umweltfaktoren bzw. meteorologischen Faktoren zu schützen, wobei ein Positionierungsabschnitt (27) hergestellt ist, der geeignet ist, eine vorbestimmte Menge der biologischen Probe aufzunehmen und als ein Hohlraum konfiguriert ist, dessen Boden zumindest teilweise aus der Erkennungsoberfläche (34) besteht, und Abdeckmittel (45) umfasst, die konfiguriert sind, den Positionierungsabschnitt (27) selektiv zu schließen und zu isolieren.

9. Vorrichtung nach Anspruch 8, **dadurch gekennzeichnet, dass** die Abdeckmittel (45) konfiguriert sind, den Positionierungsabschnitt (27) elektromagnetisch zu isolieren und hermetisch abzudichten.

10. Tragbares Diagnosekit zum Analysieren einer biologischen Probe eines menschlichen oder tierischen Subjekts, umfassend mindestens eine Diagnosevorrichtung 10 in Taschengröße nach einem der Ansprüche 1 bis 9 und eine Injektorvorrichtung, die konfiguriert ist, dem Subjekt eine therapeutische Dosis eines Arzneimittels, das insbesondere Adrenalin enthält, zu verabreichen.

11. Diagnoseverfahren zum Analysieren einer biologischen Probe eines menschlichen oder tierischen Subjekts, um eine Überempfindlichkeitsreaktion in dem Subjekt zu diagnostizieren, wobei das Verfahren Folgendes umfasst:
- Bereitstellen einer tragbaren Diagnosevorrichtung (10) in Taschengröße nach einem der Ansprüche 1 bis 9;
- Positionieren einer biologischen Probe direkt auf und in Kontakt mit der Erkennungsoberfläche (34);
- Durchführen einer Erfassung eines Probensignals (S1), wenn die biologische Probe in Kontakt mit der Erkennungsoberfläche (34) ist;
- Verarbeiten des Probensignals (S1) durch Vergleich mit einem Referenzsignal (S0), um die Überempfindlichkeitsreaktion zu diagnostizieren;
- Signalisieren des Ergebnisses der Diagnose.

12. Verfahren nach Anspruch 11, **dadurch gekennzeichnet, dass** es vor dem Verarbeiten das Durchführen einer Referenzerfassung in Abwesenheit der biologischen Probe umfasst, um das Referenzsignal (S0) zu erhalten.

13. Verfahren nach Anspruch 11 oder 12, **dadurch gekennzeichnet, dass** die biologische Probe auf der Erkennungsoberfläche (34) innerhalb eines Positionierungsabschnitts (27) positioniert wird und dass die Erfassung der Signale (S1, S0) stattfindet, sobald der Positionierungsabschnitt (27) elektromagnetisch isoliert und hermetisch abgedichtet ist, um Störungen aufgrund von Strahlung, Magnetfeldern, Luft, Wasser und Feuchtigkeit zu verhindern, die von der Umgebung außerhalb des Positionierungsabschnitts (27) kommen.

14. Verfahren nach einem der Ansprüche 11 bis 13, **dadurch gekennzeichnet, dass** das Verarbeiten das Berechnen mindestens eines Verhältnisses zwischen dem Probensignal (S1) und dem Referenzsignal (S0) ermöglicht, ausgewählt aus:
- einem Verhältnis zwischen der Intensität des Probensignals (S1) und der Intensität des Referenzsignals (SO);
- einem Verhältnis zwischen der Frequenzverschiebung des Probensignals (S1) und der Frequenzverschiebung des Referenzsignals (SO);
- einem Verhältnis zwischen der Phasenvariation des Probensignals (S1) und der Phasenvariation des Referenzsignals (S0).

15. Verfahren nach einem der Ansprüche 11 bis 14, **dadurch gekennzeichnet, dass** das Signalisieren mindestens eines von entweder dem Senden einer Notfallkommunikation über ein Telefonnetz oder ein Internetnetz an einen zuvor bestimmten Empfänger oder das Erfassen der Geolokalisierungskoordinaten der Vorrichtung (10) und das Übertragen derselben an den Empfänger mittels der Notfallkommunikation umfasst.

## Revendications

1. Dispositif de diagnostic portatif de poche pour analyser un échantillon biologique d'un sujet humain ou animal afin de diagnostiquer une réaction d'hypersensibilité chez ledit sujet, ledit dispositif comprenant un biocapteur optique plasmonique comprenant :
i) un substrat de bio-reconnaissance (13) qui présente une surface de reconnaissance (34), qui peut recevoir ledit échantillon biologique et sur laquelle des liants biochimiques (15) sont immobilisés, configurée pour se lier sélectivement à des marqueurs biochimiques (12) associés à ladite réaction d'hypersensibilité présente dans ledit échantillon biologique, formant des complexes marqueurs-liants (16) en une quantité corrélée à une réaction d'hypersensibilité chez ledit sujet, et une surface d'interface diélectrique (35), opposée à ladite surface de reconnaissance (34), dans lequel ladite surface de reconnaissance (34) présente une finition de surface comprenant une pluralité de canaux de tailles micrométriques et/ou nanométriques et lesdits liants biochimiques (15) ne sont que des anticorps anti-bêta-tryptase ;
ii) un transducteur optique plasmonique (17) couplé audit substrat de bio-reconnaissance (13) et comprenant une couche métallique (21) ayant une surface métallique d'interface (36) disposée en contact avec ladite surface d'interface diélectrique (35) définissant une région d'interface optique plasmonique (37) ;
iii) une source de rayonnement électromagnétique (18) pouvant émettre un rayonnement incident (24) vers ladite région d'interface optique plasmonique (37) ;
iv) un détecteur de rayonnement électromagnétique (19) pouvant détecter un signal optique (25) réfléchi par ladite région d'interface optique plasmonique (37), corrélé à une quantité de complexes marqueurs-liants (16) qui se forment sur la surface de reconnaissance (34) dudit substrat de bio-reconnaissance (13), et de le transformer en un signal électrique.

2. Dispositif selon la revendication 1, **caractérisé en ce que** lesdits marqueurs biochimiques (12) comprennent un bêta-tryptase.

3. Dispositif selon l'une quelconque des revendications précédentes, **caractérisé en ce que** ledit substrat de bio-reconnaissance (13) comprend une phase solide (14) de dextrane sur laquelle les liants biochimiques (15) sont immobilisés.

4. Dispositif selon l'une quelconque des revendications précédentes, **caractérisé en ce qu'il** comprend également des moyens de traitement (33) configurés pour traiter ledit signal électrique et déterminer si ladite quantité desdits complexes marqueurs-liants (16) se situe dans une plage de quantités indicative d'une réaction d'hypersensibilité chez ledit sujet.

5. Dispositif selon l'une quelconque des revendications précédentes, **caractérisé en ce qu'il** comprend des moyens de signalisation (32) configurés pour fournir à un opérateur des informations corrélées à la présence et/ou à l'intensité d'une réaction d'hypersensibilité dans l'échantillon biologique corrélée au signal électrique obtenu.

6. Dispositif selon l'une quelconque des revendications précédentes, **caractérisé en ce qu'il** comprend également des moyens de localisation (30) configurés pour détecter les coordonnées de géolocalisation dudit dispositif (10).

7. Dispositif selon l'une quelconque des revendications précédentes, **caractérisé en ce qu'il** comprend également des moyens de communication (31) configurés pour envoyer une communication d'urgence par réseau téléphonique ou réseau Internet à un destinataire déterminé précédemment.

8. Dispositif selon l'une quelconque des revendications précédentes, **caractérisé en ce qu'il** comprend également un corps contenant (26) configuré pour protéger le dispositif (10) des contraintes mécaniques et/ou des facteurs environnementaux et météorologiques dans lesquels une partie de positionnement (27) est réalisée, appropriée pour loger une quantité prédéterminée dudit échantillon biologique et configurée comme une cavité, dont le fond est constitué, au moins en partie, de ladite surface de reconnaissance (34), et des moyens de recouvrement (45) configurés pour fermer et isoler sélectivement ladite partie de positionnement (27).

9. Dispositif selon la revendication 8, **caractérisé en ce que** lesdits moyens de recouvrement (45) sont configurés pour isoler électromagnétiquement et sceller hermétiquement ladite partie de positionnement (27).

10. Kit de diagnostic portable pour analyser un échantillon biologique d'un sujet humain ou animal comprenant au moins un dispositif de diagnostic de poche 10 selon l'une quelconque des revendications 1 à 9, et un dispositif d'injection, configuré pour administrer audit sujet une dose thérapeutique de médicament, en particulier contenant de l'adrénaline.

11. Procédé de diagnostic pour analyser un échantillon biologique d'un sujet humain ou animal afin de diagnostiquer une réaction d'hypersensibilité chez ledit sujet, ledit procédé consistant à :
- fournir un dispositif de diagnostic portable de poche (10) selon l'une quelconque des revendications 1 à 9 ;
- positionner un échantillon biologique directement sur et en contact avec la surface de reconnaissance (34) ;
- effectuer une acquisition d'un signal d'échantillon (S1) lorsque ledit échantillon biologique est en contact avec ladite surface de reconnaissance (34) ;
- traiter ledit signal d'échantillon (S1), par comparaison avec un signal de référence (S0) afin de diagnostiquer ladite réaction d'hypersensibilité ;
- signaler le résultat dudit diagnostic.

12. Procédé selon la revendication 11, **caractérisé en ce qu'**il comprend, avant ledit traitement, la réalisation d'une acquisition de référence, en l'absence dudit échantillon biologique, pour obtenir ledit signal de référence (S0).

13. Procédé selon la revendication 11 ou 12, **caractérisé en ce que** l'échantillon biologique est positionné sur ladite surface de reconnaissance (34) à l'intérieur d'une partie de positionnement (27) **et en ce que** l'acquisition desdits signaux (S1, S0) a lieu lorsque la partie de positionnement (27) est isolée électromagnétiquement et scellée hermétiquement, afin d'empêcher les perturbations dues au rayonnement, aux champs magnétiques, à l'air, à l'eau et à l'humidité, provenant de l'environnement à l'extérieur de ladite partie de positionnement (27).

14. Procédé selon l'une quelconque des revendications 11 à 13, **caractérisé en ce que** ledit traitement prévoit de calculer au moins un rapport entre le signal d'échantillon (S1) et le signal de référence (S0), choisi parmi :
- un rapport entre l'intensité du signal d'échantillon (S1) et l'intensité dudit signal de référence (S0) ;
- un rapport entre le décalage de fréquence du signal d'échantillon (S1) et le décalage de fréquence du signal de référence (S0) ;
- un rapport entre la variation de phase du signal d'échantillon (S1) et la variation de phase du signal de référence (S0).

15. Procédé selon l'une quelconque des revendications 11 à 14, **caractérisé en ce que** ladite signalisation comprend au moins l'une des étapes consistant à envoyer une communication d'urgence par réseau téléphonique ou réseau Internet à un destinataire déterminé précédemment ou à détecter les coordonnées de géolocalisation du dispositif (10) et à les transmettre audit destinataire au moyen de ladite communication d'urgence.
